Europäisches Patentamt

(19) European Patent Office

Office européen des brevets

(11) EP 0 928 615 A1

# (12) EUROPEAN PATENT APPLICATION
published in accordance with Art. 158(3) EPC

(43) Date of publication:
14.07.1999 Bulletin 1999/28

(51) Int. Cl.$^6$: A61M 1/28

(21) Application number: 97924350.8

(86) International application number:
PCT/JP97/01950

(22) Date of filing: 09.06.1997

(87) International publication number:
WO 97/47337 (18.12.1997 Gazette 1997/54)

(84) Designated Contracting States:
BE DE FR GB IT NL SE

(30) Priority: 12.06.1996 JP 15093096

(71) Applicant:
A.S.A. Sangyo CO., Ltd.
Sakura-shi, Chiba-ken 582 (JP)

(72) Inventor:
SAKAI, Asahi,
A.S.A. Sangyo Co. Ltd
Sakura-shi, Chiba-ken 285 (JP)

(74) Representative:
Cresswell, Thomas Anthony
J.A. KEMP & CO.
14 South Square
Gray's Inn
London WC1R 5LX (GB)

## (54) METHOD FOR RECOVERING AND REGENERATING PERITONEAL DIALYZATE, APPARATUS THEREFOR, AND ATTACHMENTS THEREOF

(57) The present invention is a recovery and regeneration process of peritoneal dialysate for chronicle renal failure patient and an apparatus therefor and an attachment thereof.

For recycling the patient's plasma protein as osmotic agent in peritoneal dialysis liquor, the effluent is recovered and uremic toxin is removed without denaturing the plasma protein under sterile condition and concentrated to the consistency to create hyper-osmolality so as to regenerate the efficient and physiologically safe dialysate by the following way ;

The recovered liquor is concentrated with semi-permeable membrane to the consistency not so high as to be denatured and thence diluted with sterile water or physiological electrolyte solution repeatedly a few times, so that small molecule uremic toxin and excess liquor is removed to raise the osmolality. If needed, electrolyte, amino acid or other useful solute is added for obtaining hyper-osmolality.

The invention also provides the apparatus and the attachments for the process.

Figure 1

EP 0 928 615 A1

Printed by Xerox (UK) Business Services
2.16.7/3.6

## Description

FIELD OF THE INVENTION

The present invention relates to the recovery and regeneration process of peritoneal dialysate that is used for dialysis therapy of renal failure, from which uremic toxin is removed, if needed, any useful components are supplemented for its reuse and its apparatus and attachments for the process

BACKGROUND OF THE INVENTION

As the therapy for chronic renal failure disease, hemodialysis has been widely applied in the world and enteral uremic toxin absorber is used before starting dialysis.. Additionally continuous ambulatory peritoneal dialysis has been also applied and recognized to have many advantages over hemo-dialysis as it removes uremic toxin continuously through the patient's peritoneum like living kidney does, compared with hemodialysis which uses artificial semipermeable membrane, and it allows the patient receive the therapy at patient home so that he has longer free time for social activity. However peritoneal dialysis has a few problems to be improved as follows;

In peritoneal dialysis, the dialysate is infused into the patient peritoneal cavity through the implanted catheter and after a few hours dwelling or recirculating, it is drained out. This procedure is repeated a few times a day. The dialysate is made of electrolyte solution. The chronical renal failure patient is incapable to exclude uremic toxin into urine. Consequently in peritoneal dialysis therapy, uremic toxin such as urea, creatinine and uric acid are diffused out from blood vessel through peritoneum into dialysate in peritoneal cavity.

Also excess liquid must be excluded from the body like kidney does in urine. In hemo-dialysis, extracoporeal blood circulation line is kept at higher pressure against dialysate circulation line, so excess liquor in blood is squeezed out through the dialyzer membrane.

In peritoneal dialysis, such static pressure difference may not be created between blood and dialysate. Consequently hyperosmotic solution is used as dialysate in which excess liquid in the patient body is ultrafiltered through peritoneum by osmotic pressure gradient. Hyperosmotic dialysate is prepared by dissolving oncotic agent such as glucose into electrolyte solution at significantly high consistency. However it is observed recently that after long term dialysis, CAPD patients suffer from severe adverse effect due to the intake of a large quantity of glucose that is added as oncotic agent through peritoneum from the dialysate every day; such as, obesity, hyperlipemia(high triglyceride consistency in serum), arterial vessel sclerosis and worsened diabetes

In order to avoid such adverse effect, various alternative oncotic agents to replace glucose have been attempted, such as oligosaccharides, hydrolyzed polysaccharides, amino acid, or hydrolyzed protein.
However every substitute has been reported to cause new problems; disturbance of metabolism, increase in maltose consistence in serum with oligo- and polysaccharides, high cost with amino acid, caramel reaction with mixture of amino acid and sugar, allergy reaction with gelatin and hydrolyzed protein, etc, if these are used in large quantity every day..
Albumin has been recognized as most physiological osmotic agent, However a large quantity of albumin from plasma may not be collected for the source without problem in economy and anemia.

While ascite has been observed in some lever, pancreas and womb cancer patients and it contains protein in similar consistency to that in serum. For therapy of the disease, the ascite is withdrawn out of abdominal cavity, concentrated and re-infused into the patient venous vessel. In case it was returned to abdominal cavity instead of venous vessel, it was reported to improve edema. However these patients had no renal dysfunction disease, the ascites did not contain uremic toxin so that no attempt was made to remove the toxin.

The present invention aims at recovering albumin and other plasma protein from the peritoneal dialysis effluent ,removing uremic toxin ,concentrating it and reusing it as osmotic agent in place of glucose, thence resolving the physiological and clinical problems due to adverse effect of glucose that is used as oncotic agent. The key point is how to recover the said protein without denaturing out of the dialysis effluent, how to remove uremic toxin and how to accumulate it at high consistency under sterile conditions and how to reuse it.

The present invention also relates to the instrument and ancillaries for the process.

DESCRIPTION OF THE INVENTION

(Recovery and Regeneration of Peritoneal Dialysate)

The present invention relates to the process to repeat the unit procedure of concentrating the plasma protein without denaturing with semi-permeable membrane of appropriate cut-off point ( maximum permeable molecular weight ) and diluting the concentrate with physiologically efficacious liquor, so as to remove small molecular weight uremic toxin out of the dialysis effluent, and to concentrate the high molecular solute to high colloidal osmolality thence to supplement electrolyte salt and any useful components for regenerating peritoneal dialysate.

(Recovery and Regenerating Apparatus)

The apparatus of the present invention is a recovery and regenerating Apparatus, which comprises a novel draining catheter, unit devices to concentrate and to dilute peritoneal dialysate, and a novel infusing as main components.

(Attachments)

The attachments of the present invention comprises catheter, of which the middle part to implant under the abdominal skin, is in shape of single smooth cylindrical tube in which the dialysate is lead for infusion alone and draining alone respectively for improving recirculation flow of the dialysate in peritoneal cavity. The outer part and inner part of the catheter is divided in two tubes. The draining tube end is connected with reverse flow-proof valve and the infusing tube end is connected with bacteria-proof filter.

(Semi-permeable Membrane)

In the present invention, semi-permeable membrane with appropriate cut-off point is used for uremic toxin removal. Here "cut-off point" indicates the maximum molecular weight (dalton) to pass through.
Main uremic toxin, such as, urea, creatinine and uric acid are small molecular weight substance, below 200 dalton, while albumin has approximately 68,00 dalton molecular weight.
So that the cut-off point of the semi-permeable membrane of the concentrating unit (11,15) may be selected between the above two values, preferably between 500 and 30,000 to minimize the loss of albumin.
If the effluent contains substantial quantity of middle molecule (500—5,000 dalton) uremic toxin, the appropriate cut-off point would be between 5,000 and 30,000.dalton.
In case the patient has amyloidosis symptom, amyloid, for example, beta 2-micro globulin, must be removed. As the substance has 11,000 dalton molecular weight, the cut-off point should be between 15,000 and 30,000 dalton.

(Concentrating)

In concentrating procedure with semi-permeable membrane, the removing coefficient is estimated by the following formula; where the concentrating ratio by volume is C;

$$100 \times (1 - 1/C) = r$$

According to the above formula, as C increases r increases.
However if C exceeds a certain level, the protein loses the solubility and deposits irreversibly. Therefore the upper limit of C must be not so great to make the protein hard to resolve again.
The lower limit would be as low as 1.5 times (a)
Below this level, the efficiency would be too small

(Diluting)

The limit of diluting ratio would be as follows;

Lower limit is as low as 0.5 times (b). Below this level next concentrating ratio is restricted to be lower than this level, resulting in uremic toxin removal efficiency too low.
Upper limit is practically physical limit. If the diluting liquor volume exceed 1,000 liter, the regenerated liquor reservoir (20) may not be easily installed at the patient home

In order to reduce the residual quantity of uremic toxin as low as 1/10 or preferably 1/25 , the cycle of concentration/ dilution unit procedure must be repeated once or a few times more
Diluting liquor could be sterile conventional peritoneal dialysate. Typical dialysate composition is shown in Table 2.
Main electrolyte salt, for example, NaCl, $MgCl_2$, $CaCl_2$, can be mixed with lactate or bicarbonate. Instead of electrolyte solution, sterile or bacteria free pure water can be used in this invention.

After the repeated concentration and dilution a few times, the final concentrate is adjusted to preset consistency by addition of diluting liquor and/or concentrate for regeneration of dialysate within the volume not exceed that of the effluent

(Pre-filtration)

In the effluent, cell and fibrin that is deposited plasma components are contained so that this solid is to be removed with pre-filtration(6).

(Absorption)

Among the globulin fraction with molecular weight 100,000—1,500,000 dalton, auto-immune antibody, complement, and lipoprotein are contained in case of rheumatism, lupus erythematosus and myasthenia gravis . If this malignant solute becomes accumulated to a high level in the recycled dialysate, it may suppress this substance to diffuse out through peritoneum into abdominal cavity furthermore. This is not desirable and halves the advantage of CAPD therapy. In order to remove these accumulated malignant fractions, before the first concentration, semi-permeable membrane (7) with cut-off point 100,000—1,000,000 dalton can be used. Also absorption column(8) that is made of, for example, porous copolymer of methyl methacrylate /divinyl benzene, epoxy cellulose gel, aspartylphenylalanine methylester or copolymer of stylene/divinyl benzene can be used. (d)

(Protein Supplement)

Usually peritoneal dialysis is performed a few times a day with low concentration glucose containing dialysate in addition to one or two hyper-osmotic dialysate for ultrafiltration. Into these dialysate plasma protein is diffused out through peritoneum every time. In the invented process, the newly diffused out protein may compensate partly or completely the loss of plasma protein during regeneration and contributes to maintaining mass balance
However in case of the patient whose malignant globulin is to be removed, loss exceeds diffused protein. In that case, required osmolality can be obtained with amino acid, albumin or oligomer of hydrolyzed protein.

(Catheter and other attachments)

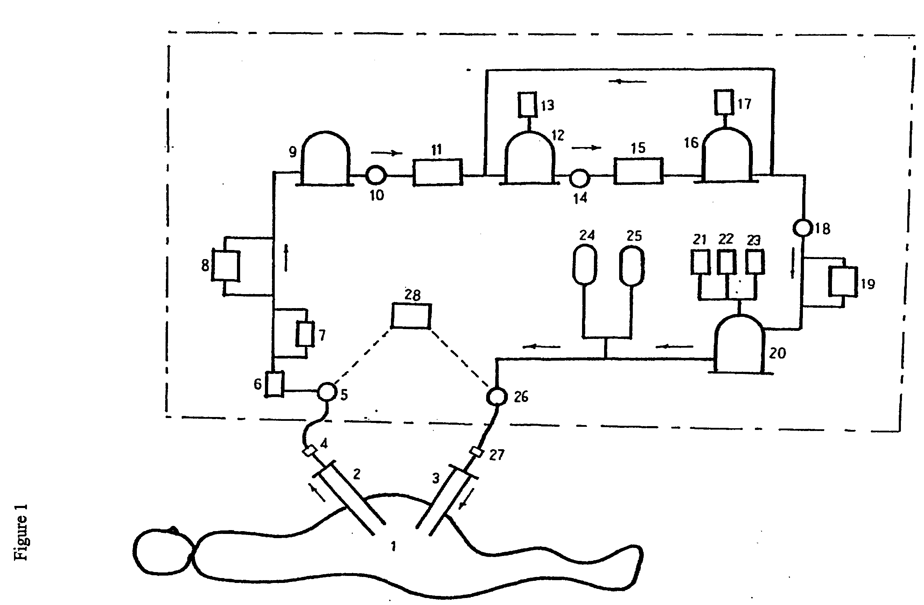

In the apparatus according to the present invention, which is illustrated in Figure 1, a new type of catheter is used which was illustrated in the preceding patent application by the same applicant as does this patent, or described in the claims 8 and 9 of this invention. The catheter consists of two tubes; i.e. infusing tube (3) and draining tube.(2) in Figure 1.
In place of the above two catheters, the invented instrument can be connected with new double lumen type of catheter directly or through reverse-proof valve(33), bacteria-proof filter (34) and pump (26) with soft polyvinyl chloride tube.

(Concentrating unit)

In the concentrating unit (11,15) of drained liquor, semi-permeable membrane in shape of hollow fiber or folded membrane plate is used in which the drained liquor is introduced under pressure. The concentrating unit membrane can be made of any synthetic polymer or animal organ membrane.

(Electrolyte liquor supply unit)

In the apparatus according to the present invention, the electrolyte liquor supply unit (13, 17) is connected to the concentrate reservoir (12, 16) or the exit of the concentrating unit (11, 15). The material for this unit is not restricted as far as it does not denature water or electrolyte.
Supply of electrolyte liquor can be made either gravity or pump. The electrolyte liquor supply unit can be soft bag made of polyvinylchloride with valve or any other material that does not denature the liquor. As described, a pair of concentrating unit (11) and the electrolyte supply unit (13) can be repeatedly connected in a series,( 15, 17) if needed. Otherwise the drained liquor can be recycled through a pair of the concentrating unit and electrolyte supply unit by connecting the exit of the electrolyte supply unit with the entrance of the concentrating unit and re-circulating with pump. The final exit of the above unit is connected with the regenerated liquor reservoir (20) then the exit of the reservoir is connected with pump (26) and the infusing catheter (3) through soft polyvinyl chloride tube.

(Filtration)

As described in Claim6, before the first concentration (11) , solid suspension such as fibrin are removed with the pre-filter (6) of which the pore size is below 100 nanometer. If needed, a large molecule globulin is removed with the semi-permeable membrane filter (7) of which cut-off point is between 100,000 and 1,000,000 dalton

(Automation system)

The invention in Claim 8 is automation system, which comprises automated regeneration system of peritoneal dialysate (g) andAutomated dialysate infusing and draining system (f).

The system (f) has the function to drain the dialysate from the patient abdominal cavity , that is stored for a few hours , through the draining catheter then to infuse the regenerated dialysate through the infusing catheter by opening and closing the valves and switch on and off the pumps according to the preset program in the satellite computer (40) or the order from the host computer(41).
Such an instrument is called "autocycler". Hyper-osmotic dialysate that is regenerated in the invention can be used in daytime or at night.

After keeping the effluent in the reservoir (9), the system (g) has the function to regenerate the dialysate, i.e. concentration, dilution and consistency adjustment, automatically in daytime, when the patient leaves away from the instrument for daily life, from the effluent recovered at night by the controller(42) according to preset program in the satellite computer (40) or the order from the host computer (41)

(Catheter)

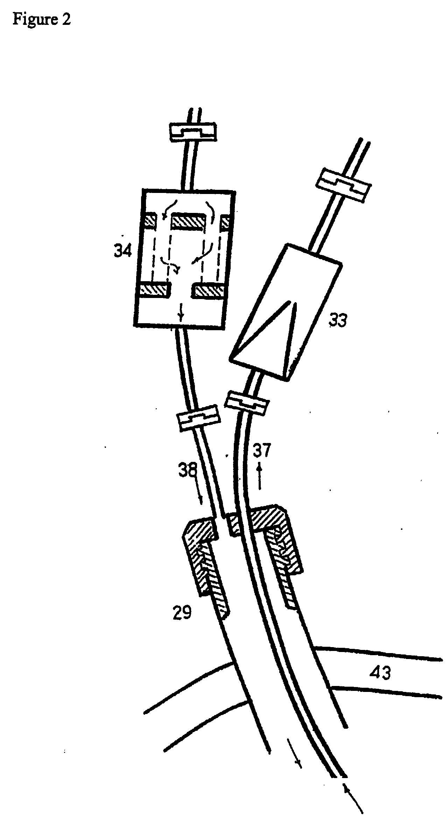

The new catheter that is illustrated in Figure 2 is used in place of two catheters(2 and 3) illustrated in Figure 1. The implanted part in human ventral lateral tunnel of the catheter is in shape of a single tube for reducing bacteria invasion along the tunnel. The catheter comprises a con-centric double lumen tube(29), of which one(37) leads the liquor drain alone, the other(38) infusion alone.

The former end is connected with reverse flow-proof valve(33), while the latter is connected with bacteria-proof filter(34) and male/female connection terminal.

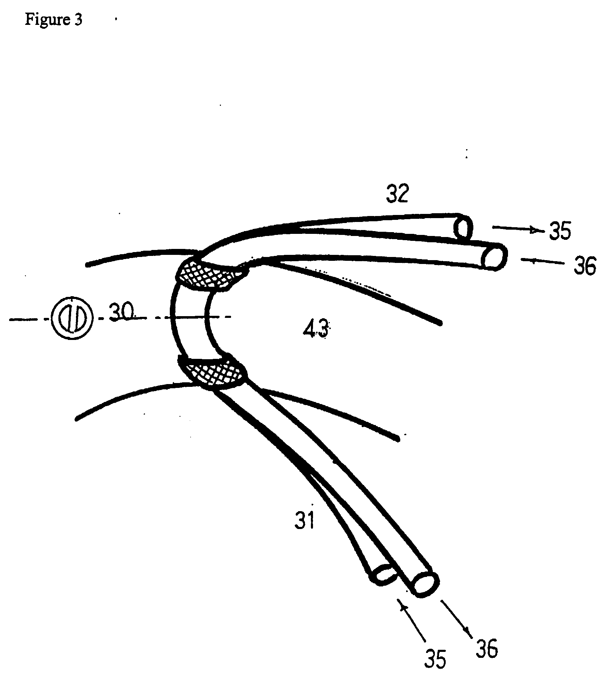

The catheter illustrated in Figure 3 .is in the shape of a single cylindrical tube in the middle part(30) in which two semicircle lumens lead the dialysate flow inward alone(36) and outward alone (35) respectively. The inner part and outer part are in the shape of divided two tubes.

(Other attachments)

Attachments described in Claim 11 are the reverse flow-proof valve(33) connected with the draining catheter exit(37) end so that it prevents flow back in of the liquor that flow out of the body once and bacteria-proof filter(34) of which membrane cut-off point is below 50 nanometer, connected with the infusing catheter entrance(38) that prevent bacteria invasion These attachments are exchanged by nurse once every month or two when the patient visits hospital.

(Use of the regenerated dialysate and the conventional one)

For the first few weeks after the invented instrument is installed at the patient's home, peritoneal dialysis is performed with conventional dialysate that contain glucose as osmotic agent while plasma protein is recovered from the effluent and accumulated in the reservoir until the quantity of protein reach to the sufficient level to provide 2 to 8 liter of hyper-osmolar dialysate .of which plasma protein consistency is greater than that of plasma, 6 to 8 gram/deciliter.
Then dialysis with the regenerated dialysate is started. After the start, the loss of protein through the regeneration process may be compensated with the new diffused out protein approximately 9 g/liter from the patient bbdy through peritoneum, except in case of specific complication, and maintain mass balance with help of small quantity of amino acid addition. .

(Conventional peritoneal dialysis with glucose containing dialysate)

Table 1 indicates dialysate composition, osmolality, ultrafiltration volume after dwelling in peritoneal cavity and energy intake based on glucose absorption through peritoneum from dialysate for each glucose content that is added as oncotic agent in dialysate at each osmotic pressure level
Typical example of usage per day is;

One bag of 4,25% dextrose (one molecule hydrate glucose)

and three bags of 1.5% dextrose or

two bags of 2.5% dextrose and two bags of 1.5% dextrose

With this prescription, 1 to 2 liter of ultrafiltration is gained, which is almost equivalent to healthy person's urine volume per day.

When the regenerated dialysate by the present invention is used, conventional 1.5-% glucose containing dialysate can be used. The regenerated hyper-osmolar dialysate take place of conventional 4.25% or 2.5% glucose dialysate.

Table 1

| Dextrose Conc. % | Osmolality mOsm/l | Ultrafiltration Volume ml in 3 hours | Energy Intake kcal / 2 liter |
|---|---|---|---|
| 1.5 | 346 | 150 | 80 |
| 2.5 | 396 | 320 | 140 |
| 4.25 | 485 | 900 | 280 |

BRIEF DESCRIPTION OF DRAWINGS

Figure 1    Peritoneal Dialysate Regeneration System
Figure 2    Catheter of the Present Invention
Figure 3    Another Catheter

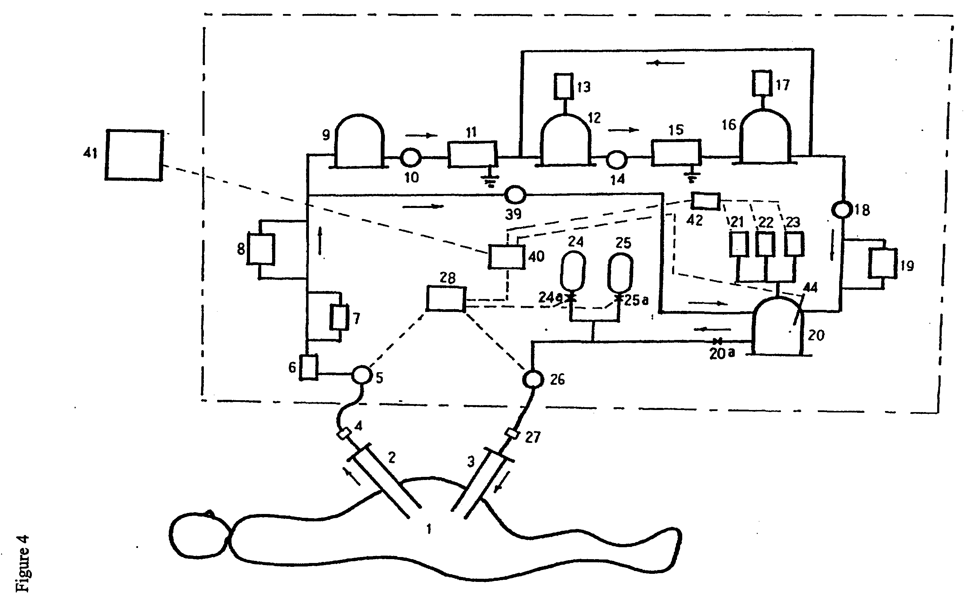

Figure 4    Computerized Regeneration System

Mark Number

[0043]

1--------- Abdominal Cavity
2-------- Draining Catheter
3-------- Infusing Catheter
4-------- Connector
5-------- Pump
6------- Pre-filter
7------- Filter
8------- Absorption Unit (1)
9------- Effluent Reservoir
10------ Pump
11------ Concentrating Unit (1)
12------- Concentrate Reservoir
13------- Electrolyte Liquor Supply Unit (1)
14------- Pump
15------- Concentrating Unit (2)
16------- Concentrate Reservoir (2)
17------- Electrolyte Liquor Supply Unit (2)
18------- Pump
19------- Absorption Unit (2)
20------- Regenerated Liquor Reservoir
20a------ Regenerated Liquor Reservoir Valve
21------- Amino Acid Supply Unit
22------- Electrolyte Concentrate Supply Unit (1)
23------- Electrolyte Concentrate Supply Unit (2)
24------- Manufactured Dialysate Bag (1)
24a------ Manufactured Dialysate Valve

25------- Manufactured Dialysate Bag (2)
25a------ Manufactured Dialysate Valve
26------- Pump
27------- Connector
28------- Controller
29------- Concentric Double Lumen Catheter
30------- Ventral Lateral Implanted Part
31------- Abdominal Cavity Inside Part
32------- Outer Part
33------- Reverse Flow-Proof Valve
34------- Bacteria Invasion-Proof Filter
35------- Draining Route
36------- Infusion Route
37------- Draining Route
38------- Infusion Route
39------- Pump
40-------- Computer
41------- Host Computer
42------- Controller
43------- Ventral Lateral
44------- Mensuration Meter

MOST PREFERRABLE EMBODYMENT FOR CARRYING OUT THE INVENTION

Example of regenerated liquor composition and operation procedure of the apparatus is described as follows;

(Regenerated Dialysate Composition)

The composition of the dialysate must be adjusted to minor extent to individual patient condition.,Hyper-phosphatemia, Hyper-calcemia, Hyper-magnesemia, Acidosis or Alkalosis, however most of the cases, similar composition to those listed in Table 2 can be used

Table 2

| ( unit: mEq /l ) | | | | | |
|---|---|---|---|---|---|
| Trade Name | $Na^+$ | $Ca^{++}$ | $Mg^{++}$ | $Cl^-$ | Lactate |
| Dianeal | | | | | |
| PD 1 | 132 | 3.5 | 1.5 | 102 | 35 |
| PD 2 | 132 | 3.5 | 0.5 | 96 | 40 |
| PD 4 | 132 | 2.5 | 0.5 | 95 | 40 |
| Perisate | 132 | 4.0 | 1.0 | 102 | 35 |
| Perisate L Ca | 132 | 2.3 | 1.0 | 98.3 | 37 |
| Peritoliq | 135 | 4.0 | 1.5 | 105.5 | 35 |
| Gambrosol | 132 | 3.5 | 0.5 | 96 | 40 |
| Gambrosol SKG | 132 | 2.0 | 0.5 | 94.5 | 40 |
| Gambrosol TCD | 135 | 2.5 | 0.5 | 98 | 40 |

(Osmotic Pressure of Regenerated Dialysate )

Blood contains 6 to 8 gram/deciliter protein and has approximately 290mOsm/l osmotic pressure. So that for ultrafiltration, hyper-osmolar dialysate must containat minimum 8 gram/deciliter, preferably 10 gram/deciliter protein up to solubility concentration at maximum. In case needed amino acid is to be added.

The osmolality of the regenerated dialysate is to be between 300 and 500 mOsm/l.

(Usage of Regenerated Dialysate )

The selection of the osmolality for each dialysis cycle is widely variable to adjust to patient daily life. Typical example of dialysate osmolality is shown below in two cases;

| Case 1 | | | |
|---|---|---|---|
|  | Storage Period hours | Osmolality mOsm/l | Use of Autocycler |
| 1 Daytime | 14 | 330 | not used |
| 2 Night | 3 | 400 | used |
| 3 Night | 4 | 330 | used |
| 4 Night | 3 | 400 | used |
| Case 2 | | | |
|  | Storage Period (hours) | Osmolarity (mOsm/l) | Use of Autocycler |
| 1 Daytime | 5 | 400 | not used |
| 2 Daytime | 8 | 350 | not used |
| 3 Night | 5 | 400 | used |
| 4 Night | 6 | 350 | used |

(Regeneration Procedure)

(1) Before sleeping at night the patient connects his catheter with the instrument, and drains the dialysate. The effluent is filtered with the pre-filter( 6 and 7) and stored in the reservoir (9). The volume every time would be approximately 2 to 3 liters.
(2) Effluent in daytime is also stored in the reservoir(9).
(3) Whole liquor volume per day, 9 to 10 liter is concentrated down to below 2 liters. The filtrate, 7 to 8 liters is discarded.
(4) The concentrate is diluted with 8 liter of diluting liquor supplied from water or electrolyte supply unit (13), then concentrated down to below 2 liter in the concentrating unit (15).
One cycle of this operation reduces the residual quantity of small molecule solute approximately down to 1/5. One more cycle reduces down to 1/25.
By more times operation cycle with smaller dilution ratio, the removal efficiency is improved at same whole diluting liquor volume usage.
(5) If the patient has complication symptom, such as amyloidosis, his drained dialysate is passed through the absorption unit(8).
(6) Into the final reservoir(20), if needed, amino acid is added.
(7) Also electrolyte concentrate is added to the reservoir(20) to adjust electrolyte consistency of the regenerated dialysate.
(8) The capacity of the final reservoir (20) is to be 10 liter or more
(9) The invented instrument is equipped with auto cycling devices, pumps and valves, which is to be opened or closed by the preset computer program in the controller (28).

(Control system)

(10) The final reservoir(20) is equipped with liquor level meter, thermometer, specific densitometer, osmotic pressure meter, electrolyte consistency meter, electro-conductometer, or any other mensuration meters(44) and monitored data is memorized in the computer (40) or sent to the host-computer (41) located in hospital or control center through the public line. According to doctor's prescription, any additional order is transferred from the host-computer back to the amino acid supply unit (21), concentrate electrolyte supply units(22 and 23 ) through the controller (42). In daytime when the patient is away from the instrument for daily life, the instrument regenerate the dialysate automatically.

At night the instrument works as autocycler to infuse and drain the dialysate according to the preset program in the computer (40) or the order from the host-computer (41) according to doctor's prescription, by opening and closing the valves (24a, 25a)and switching on and off the pumps ( 5, 26) through the controller (28)

INDUSTRIAL APPLICABILITY

By the process and apparatus of the present invention, plasma protein is recovered from the effluent and reused as osmotic agent in place of glucose, consequently the adverse effect caused by glucose such as obesity, hyper-lipemia, arteriovessel sclerosis, and diabetes, can be avoided.

Dialysis therapy, i.e. removal of uremic toxin and ultrafiltration, can be achieved without problem.

By autocycler function, the patient is free from exchange operation in daytime and reduction of dwelled dialysate in the abdominal cavity make patient comfortable so that his quality of life can be improved. Also with the ancillaries, bacteria invasion is prevented so that infection rate is reduced. Lesser peritonitis and with no glucose adverse effect, peritoneum function can be maintained much longer.

With host computer located in hospital or control center, patient can be remote monitored and controlled so as to be free from complicated instrument operation. Both safeties of home therapy and patient quality of life can be improved.

**Claims**

1. A regeneration process of dialysate that is drained from peritoneal cavity of human body through implanted catheter and recovered which comprises

   (a) a procedure to concentrate the recovered peritoneal dialysis effluent with semipermeable membrane by one and a half times at minimum and by the extent that the consistency of the organic solute becomes not too high to be easily resolved again at maximum.
   (b) a procedure to dilute the concentrate with bacteria-free or sterile electrolyte solution by 0.5 times of the concentrate volume at minimum and by 1,000 liter at maximum once or a few times of (a) and (b) procedure repeatedly, thence
   (c) a procedure to adjust the electrolyte solution volume to add at the final dilution in order not to exceed the effluent volume

2. The recovery and regeneration process according to claim 1, wherein the aforementioned concentration procedure (a) performed with semipermeable membrane, of that maximum permeable molecule lies between 500 to 30,000 dalton.

3. The recovery and regeneration process according to claim 1 or 2, in which amino acid, albumin, or hydrolyzed protein oligomer or mixture of these substances is added to the liquor in the final dilution procedure, thence electrolyte consistency is adjusted.

4. The recovery and regeneration process according to claim 1 , 2 or 3which includes absorption procedure (d) to remove a part of solute in prior to the first concentration and/or after the last dilution procedure.

5. The recovery, regeneration and infusion apparatus of peritoneal dialysate that is infused into human peritoneal cavity, and immediately or after being dwelled or re-circulated for a certain period drained out, which includes at least a drain catheter (2), devices to concentrate the recovered effluent (11), a devices to supply electrolyte solution (13), of which one a pair of a device(11) and a device(13) or more than two sets of these are connected in a series repeatedly, and an infusion catheter (3).

6. The recovery and regenerating apparatus according to claim 5 which includes a pre-filter (6) of maximum pore size below 100 nanometer and a filter(8) of maximum permeable molecule lies between 100,000 to 1,000,000 dalton before the first concentrating unit (11).

7. The recovery and regenerating apparatus according to claim 5 or 6, which includes concentrating units (11, 15) of which semi-permeable membrane's maximum permeable molecule lies between 500 and 30,000.

8. The recovery and regenerating apparatus according to claim 5, 6 or 7 which includes a control system (f) to open and close a valve of the dialysate reservoir(20a) and valves of fresh dialysate containers (24a, 25a) following the order from a host-computer (41) in which time program of dialysis cycle and infusing dialysate volume are preset, through a computer (40) and a controller (28). and / or a control system (g) to transfer the data that is obtained by measure (44) at a dialysate reservoir (20) and its exit, to a host-computer (41) through a computer (40) and to supplement the estimated volume of amino acid (21) and concentrated electrolyte solution (22, 23) following host-computer's order(41) through a computer (40) and a controller (42).

9. The catheter used in the recovery and regeneration apparatus according to claim 5,6,7 or 8, of which the inner part comprises con-centric double lumen tube and of which one is used for infusion and the other for draining respectively and connected with the recovery and regeneration apparatus, in place of a single draining catheter (2) and a single infusing catheter (3).

10. The catheter used in the recovery and regeneration apparatus according to claim 5,6,7 or 8, of which the middle part that is implanted in patient's ventral lateral is in shape of a single smooth cylindrical tube in which two semicircle lumens leads liquor infusing through one lumen and draining through the other lumen respectively and in shape of two divided tubes in inner(31) and outer(32) parts, in place of a single draining catheter (2) and a single infusing catheter (3).

11. The catheter according to claim 5,6,7,8 or 9 of which exit of a draining tube is connected with reverse flow-proof valve(33) and of which the entrance of a infusion tube is connected with bacteria-proof filter that comprises semi-permeable membrane of maximum pore size below 50 nanometer.

Figure 1

Figure 2

Figure 3

Figure 4

## INTERNATIONAL SEARCH REPORT

| International application No. |
| --- |
| PCT/JP97/01950 |

**A. CLASSIFICATION OF SUBJECT MATTER**

Int. Cl$^6$ A61M1/28

According to International Patent Classification (IPC) or to both national classification and IPC

**B. FIELDS SEARCHED**

Minimum documentation searched (classification system followed by classification symbols)

Int. Cl$^6$ A61M1/28, A61M1/14

Documentation searched other than minimum documentation to the extent that such documents are included in the fields searched

Jitsuyo Shinan Koho          1926 - 1996     Jitsuyo Shinan Toroku
Kokai Jitsuyo Shinan Koho    1971 - 1997     Koho          1996 - 1997
Toroku Jitsuyo Shinan Koho   1994 - 1997

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| P | JP, 08-337590, A (Terumo Corp.), December 24, 1996 (24. 12. 96), Claim 1 (Family: none) | 1 - 7 |
| A | JP, 04-327857, A (Nissho Corp.), November 17, 1992 (17. 11. 92), Claim 1; Fig. 1 (Family: none) | 9 |

☐ Further documents are listed in the continuation of Box C.   ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | |
| | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| September 2, 1997 (02. 09. 97) | September 17, 1997 (17. 09. 97) |

| Name and mailing address of the ISA/ | Authorized officer |
| --- | --- |
| Japanese Patent Office | |
| Facsimile No. | Telephone No. |

Form PCT/ISA/210 (second sheet) (July 1992)